# FASCICULE DE BREVET EUROPEEN

(11) **EP 0 829 719 B1**
(45) Date de publication et mention de la délivrance du brevet: **05.12.2001**
(21) Numéro de dépôt: 97401956.4
(22) Date de dépôt: 20.08.1997
(51) Int. Cl.: G01N 33/24, G01N 31/12

(54) **Méthode et dispositif d'évaluation d'une caractéristique de pollution d'un échantillon de sol**
Verfahren und Vorrichtung zur Auswertung einer Kontaminationskarakteristik einer Bodenprobe.
Method and device for avaluating a pollution characteristic of a soil sample.

(30) Priorité: 12.09.1996 FR 9611582
(43) Date de publication de la demande: 18.03.1998
(73) Titulaire: INSTITUT FRANCAIS DU PETROLE, 92852 Rueil-Malmaison Cedex (FR)
(72) Inventeur: Lafargue, Eric, 1, Résidence "Le Val d'Eglantines", 78420 Carrieres Sur Seine (FR); Pillot, Daniel, 75017 Paris (FR); Ducreux, Jean, 78380 Bougival (FR); Marquis, François, 95680 Montlignon (FR)

(56) Documents cités:
- EP-A- 0 691 540
- US-A- 4 229 181
- US-A- 4 352 673
- US-A- 4 519 983
- ESPITALIE J ET AL: "IMPROVE EXPLORATION SUCCESS USING NEW SOURCE ROCK ANALYZER" WORLD OIL, vol. 217, no. 4, 1 avril 1996, page 111/112 XP000596488
- J. ESPITALI ET AL.: "Méthode rapide d'analyse géochimique des déblais en suivi de forage" PETROLE ET TECHNIQUES, no. 283, octobre 1981, PARIS , FR, pages 99-103, XP002031865

## Description

La présente invention concerne une méthode et un dispositif pour déterminer au moins une caractéristique de pollution d'un sol naturel potentiellement ou réellement contaminé par des produits polluants, notamment des hydrocarbures et/ou dérivés.

Pour une meilleure compréhension, on rappelle que:
- au cours d'un déversement accidentel ou chronique (par exemple, rupture d'un pipe-line, perte d'étanchéité d'un stockage, par exemple une cuve ou une citerne, ou après abandon d'anciens sites industriels, une certaine quantité de composés hydrocarbonés. peuvent s'infiltrer dans les sols, ce qui donne lieu à une pollution de tout ou partie dudit sol,
- la connaissance du type de polluant (essence, kérosène, gazole, lubrifiant, dérivés chlorés,...) ainsi que la connaissance de l'extension dans l'espace et dans le temps de la pollution sont d'un grand intérêt pour les responsables chargés d'études de diagnostics, d'impact sur l'environnement et de la réhabilitation des sols pollués. Il est en effet connu que les techniques de réhabilitation employées dépendent du type de produits polluants.

De ce fait, il est très important de déterminer rapidement la nature des produits polluants et la quantité de sol à traiter qui dépend directement de l'extension de la pollution tant en profondeur qu'en surface. La détermination du degré de pollution d'un sol permet d'évaluer les volumes de terrain à traiter, de déterminer les meilleures méthodes de traitement, et ainsi les coûts correspondants à la mise en oeuvre.

Il apparaît alors que l'analyse systématique d'échantillons de sols potentiellement ou réellement pollués permet d'établir rapidement un diagnostic portant sur la nature et l'étendue de la pollution ainsi que sur les risques essentiels associés (contamination d'une nappe phréatique, par exemple).

La connaissance de tels renseignements permet d'optimiser les opérations de réhabilitation des sites contaminés dès le stade du diagnostic. Sans les éliminer complètement, on limite les analyses de laboratoire longues et coûteuses, à un minimum nécessaire en complément des renseignements collectés systématiquement à l'aide de la méthode selon la présente invention.

On connaît la technique ROCK-EVAL développée par la demanderesse et décrite notamment dans les documents US-A-4153415, 4229181, 4352673, 4519983, ainsi que le brevet français FR-A-2 722 296. Cette méthode qui est rapide, pratiquement automatique, a été développée pour la caractérisation des roches mères ou des roches réservoirs, ainsi que des hydrocarbures qu'elles contiennent.

Cependant, cette méthode et le dispositif ne sont pas adaptés pour caractériser de façon précise les différentes coupes hydrocarbonées que peuvent contenir des sols pollués par de tels produits.

Ainsi, la présente invention est un perfectionnement de la technique ROCK-EVAL permettant de caractériser de façon précise des polluants, notamment de type hydrocarbures et/ou dérivés (composés chlorés, soufrés,...) contenus dans un sol pollué.

La présente invention concerne un méthode améliorée permettant l'évaluation rapide d'au moins une caractéristique de pollution de sols naturels à partir d'un échantillon dudit sol chauffé, dans un premier temps en atmosphère non oxydante puis, dans un second temps, en atmosphère oxydante selon plusieurs étapes d'élévation de température. La méthode comporte au moins les étapes suivantes :
a) on élève rapidement la température de l'échantillon à une première valeur de température T₁ comprise entre 80 et 120°C et pendant une durée déterminée (t₁-t₀),
b) à partir de la première valeur de température, on élève la température de l'échantillon jusqu'à une seconde valeur de température T₂ inférieure à 200°C selon un gradient de température compris entre 2 et 30°C/min et on demeure à cette température T₂ pendant une durée déterminée (t₃-t₂),
c) on élève la température de la seconde valeur à une troisième valeur T₃ de température inférieure à 500°C, selon un gradient de température compris entre 10 et 40°C/min,
d) on élève la température de l'échantillon de la troisième valeur à une quatrième valeur T₄ au plus égale à 850°C,
e) on détermine à partir des quatre étapes précédentes, au moins quatre grandeurs Q₀, Q₁, Q₂, Q₃, représentatives de la nature et de la quantité des composés hydrocarbonés contenus dans ledit échantillon,
f) après l'élévation de température à la quatrième valeur T₄, on brûle, en atmosphère oxydante, les résidus dudit échantillon depuis une température supérieure à 350°C jusqu'à une température au plus égale à 850°C, selon un gradient de température compris entre 20 et 50°C/min,
g) on détermine une quantité Q₄ représentative de la quantité de carbone organique résiduel après les quatre étapes de chauffe,
h) on déduit des cinq grandeurs Q₀, Q₁, Q₂, Q₃ et Q₄ au moins une caractéristique de pollution dudit échantillon.

On peut évaluer la quantité Q de polluant selon la formule suivante :

Q = Q₀ + Q₁ + Q₂ + Q₃ + kQ₄

avec k de 10 à 11,5.

On peut déterminer au moins une fonction liant au moins une grandeur parmi le groupe constitué par Q₀, Q₁, Q₂, Q₃, et Q₄, et on peut distinguer le type "a", "b", "c", ou "d" du polluant en fonction d'au moins une valeur spécifique de la fonction pour un échantillon donné.

On peut distinguer le type "a", "b", "c", ou "d" du polluant à l'aide d'au moins l'un des rapports spécifiques suivants :
- pour un polluant de type "a", le rapport Q₀/(Q₀+Q₁+Q₂) est compris entre 0,5 et 1 et le rapport (Q₃+Q₄)/(Q₀+Q₁+Q₂) est voisin de zéro,
- pour un polluant de type "b", le rapport Q₀/(Q₀+Q₁+Q₂) est compris entre 0,05 et 0,5 et le rapport (Q₃+Q₄)/(Q₀+Q₁+Q₂) est voisin de zéro,
- pour un polluant de type "c", le rapport Q₀/(Q₀+Q₁+Q₂) est voisin de zéro, et le rapport (Q₃+Q₄)/(Q₀+Q₁+Q₂) est compris entre 0 et 5,
- pour un polluant de type "d", le rapport Q₀/(Q₀+Q₁+Q₂) est voisin de zéro, et le rapport (Q₃+Q₄)/(Q₀+Q₁+Q₂) est supérieur à 10.

L'invention concerne également un dispositif pour l'évaluation d'au moins une caractéristique de pollution de sols naturels par des composés hydrocarbonés, à partir d'un échantillon desdits sols placé dans une nacelle, ledit dispositif comportant un premier moyen de chauffe dudit échantillon sous atmosphère non oxydante, des moyens de mesure de la quantité des composés hydrocarbonés dégagés à la suite de l'introduction de l'échantillon dans ledit premier moyen de chauffe, des moyens de transfert de l'échantillon dans un second moyen de chauffe sous atmosphère oxydante, des moyens de mesure de la quantité de CO₂ contenue dans les effluents évacués des deux moyens de chauffe, lesdits moyens de mesure du CO₂ comportant une cellule de mesure en continu du CO₂ à chaque instant du cycle de chauffe du premier et du second moyen de chauffe et comportant des moyens de mesure de la quantité de CO contenue dans les effluents évacués par les deux moyens de chauffe. Le dispositif comporte des moyens de détermination du type de polluant, par exemple des moyens de calcul d'une fonction particulière liant au moins une des grandeurs Q₀, Q₁, Q₂, Q₃, et Q₄.

Le dispositif peut comporter des moyens de contrôle de la température des nacelles en attente d'introduction dans lesdits moyens de chauffe.

Les moyens de contrôle de la température peuvent comporter l'un au moins des éléments suivants: une ventilation, une circulation d'un fluide réfrigérant, une isolation.

Les nacelles peuvent comporter des moyens d'obturation.

Le dispositif peut comporter des moyens d'ouverture des nacelles avant introduction dans les moyens de chauffe, par exemple sur le bras de chargement et transfert, ou sur le piston de chargement de la nacelle dans le four.

L'invention sera mieux comprise et ses avantages apparaîtront plus clairement à la lecture de la description d'exemples, nullement limitatifs, illustrés par les figures annexées, parmi lesquelles :
- la figure 1 représente schématiquement la composition d'un sol pollué par différentes coupes hydrocarbonées,
- la figure 2 décrit les moyens de mise en oeuvre de la méthode selon l'invention,
- la figure 3 montre les différentes étapes de chauffe de l'échantillon en fonction du temps,
- la figure 4 montre la forme d'un exemple d'enregistrement des quantités Q,
- les figures 5A, 5B, 5C et 5D illustrent un exemple de caractérisation des produits polluants.

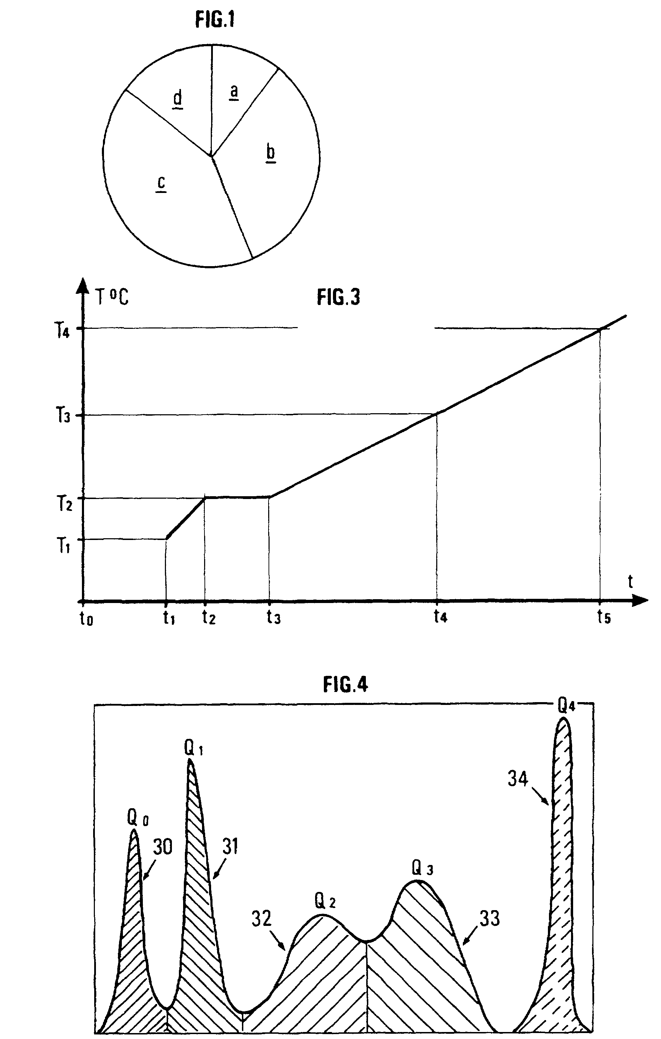

La figure 1 illustre schématiquement un exemple de composition d'un échantillon de sol naturel contaminé par différents types de produits polluants.

On peut distinguer au moins les quatre zones principales suivantes:
- la zone "a" représente majoritairement la quantité de composés hydrocarbonés légers, à point d'ébullition inférieur à 230°C, tels que les coupes de type essence, kérosène, et/ou les composés halogénés légers. Dans cette zone, le nombre d'atomes de carbone est majoritairement inférieur à 12. Dans la présente description, les composés de cette zone seront dénommés comme appartenant au type "a";
- la zone "b" représente majoritairement la quantité de composés hydrocarbonés, à point d'ébullition compris essentiellement entre 230°C et 400°C, tels que les coupes de type "gazole" et ou les composés halogénés de type polychlorobiphényl, etc.... Dans cette zone, le nombre d'atomes de carbone est majoritairement compris entre 13 et 25. Dans la présente description, les composés de cette zone seront dénommés comme appartenant au type "b";
- la zone "c" représente majoritairement la quantité de composés hydrocarbonés, à point d'ébullition compris entre 400 et 550°C, tels que les coupes pétrolières lourdes, les huiles lubrifiantes, etc.... Dans cette zone, le nombre d'atomes de carbone est majoritairement compris entre 25 et 40. Dans la présente description, les composés de cette zone seront dénommés comme appartenant au type "c";
- la zone "d" représente majoritairement la quantité de composés hydrocarbonés, à point d'ébullition sensiblement supérieur à 550°C, tels des résidus de distillation, et dont le nombre d'atomes de carbone est essentiellement supérieur à 40. Dans la présente description, les composés de cette zone seront dénommés comme appartenant au type "d".

Chacune des zones a, b, c, et d, est donc caractérisée par des composés principaux en quantité majoritaire selon la définition ci-dessus. Il est cependant possible que des produits polluants contenant en plus d'autres composés de température d'ébullition sensiblement différente ou d'un nombre de carbone différent de ce qui est défini pour une zone considérée, soient classés dans ladite zone car ne modifiant pas la caractérisation selon l'invention.

L'objet de la méthode selon la présente invention est de permettre de distinguer, et dans le même temps, de quantifier les différents types de polluants contenus dans un échantillon de sol, compte tenu de leur appartenance à l'une des zones définies ci-dessus. Au cours d'une campagne de diagnostic d'un site pollué, on prélève des échantillons de sol en différents endroits et à différentes profondeurs pour les caractériser par le moyen de la présente méthode dans le dispositif correspondant. Les résultats de caractérisation vont permettre l'établissement de "profils" de pollution du sol en différenciant ainsi les terrains pollués des terrains préservés, ainsi que la nature et la gravité de la pollution.

La figure 2 décrit le dispositif permettant la mise en oeuvre selon l'invention. Il est composé d'un automate qui effectue les mesures et d'un PC ("Personal Computer") qui pilote l'automate, sert d'interface avec d'autres calculateurs, gère les analyses, permet la visualisation des résultats en temps réel et qui utilise des logiciels de contrôle et de tests.

Le dispositif de mesure comporte deux micro fours, un passeur d'échantillons qui les alimente en nacelles et un système d'analyse composé d'un ou de plusieurs détecteurs spécifiques, par exemple un détecteur à ionisation de flamme (FID) ou un détecteur à capture d'électrons ECD, et de cellules à infrarouge (IR). Ces éléments sont connectés à une électronique et à un circuit de fluides gérés par le PC et par le software de l'automate.

Sur la figure 2, la référence 1 désigne l'ensemble de chauffe adapté à la pyrolyse de l'échantillon 2 placé dans une nacelle 3 portée par un piston 4. Des moyens de déplacements 5 de la nacelle introduisent l'échantillon dans l'espace intérieur 6 du four. Les moyens de déplacements peuvent être des vérins à commande pneumatique, hydraulique ou électrique. La référence 7 schématise le conduit d'amenée du fluide vecteur de balayage des produits pyrolysés dans le four. Ce fluide (azote ou hélium) balaye l'échantillon en passant par le piston. Des moyens de distribution (non représentés) conduisent le fluide vecteur à la partie supérieure du four pour effectuer une purge en rétro-balayage de l'intérieur du four lors du recul du piston, par exemple en fin de pyrolyse pour transférer l'échantillon et/ou pour charger un autre échantillon. En effet, l'influence de l'oxygène sur les dépôts organiques des parois du four de pyrolyse peut générer des composés oxygénés CO₂ et CO qui sont gênants pour l'analyse.

Une sonde de température 8 mesure la température au niveau du fond de la nacelle, donc très proche de l'échantillon. Une autre sonde de température 9 a son point de mesure dans la paroi du four, au niveau de la position haute de la nacelle, position correspondant au point de chauffe optimal. La programmation en température du four est effectuée de préférence à l'aide de la sonde 8, ce qui permet un bon contrôle et une bonne connaissance de la température de pyrolyse de l'échantillon. La sonde de température 9 est utilisée pour contrôler la température du four 1 lorsque le four est ouvert et que le piston 4 est descendu pour extraire la nacelle 3 et la remplacer par une autre. Ainsi, la température du four 1 peut être maintenue à une valeur proche de la valeur déterminée pour la pyrolyse suivante ce qui permet d'éviter trop de perte thermique.

L'ensemble de chauffe la est identique en tout point à l'ensemble de chauffe 1. Cet ensemble la est dévolu à l'opération d'oxydation d'un échantillon, généralement après pyrolyse. Les éléments identiques sont indexés "a". Il faut noter que le fluide injecté par le conduit 7a est dans ce cas de l'air.

Les ensembles de chauffe 1 et 1a ont tous les deux des moyens de régulation de la température qui permettent une programmation du gradient de température, laquelle pouvant atteindre et même dépasser 850°C.

La référence 10 désigne le détecteur FID à flamme ionisante délivrant un signal S représentatif des quantités de produits hydrocarbonés dégagés de l'échantillon en cours de chauffe. La flèche 11 symbolise le transfert du signal S vers les moyens de digitalisation. Le détecteur à ionisation de flamme FID doit tenir aux températures élevées d'où la nécessité pour lui de contenir des joints supportant ces conditions sans fluer ni désorber des produits pouvant faire dévier la ligne de base.

Sa linéarité et sa sensibilité associées à une dérive de la ligne de base très faible sont les gages d'une analyse de grande précision des hydrocarbures.

Le signal analogique sera digitalisé et lissé avec le maximum de points qui est fonction de la vitesse de programmation.

A la place du FID, on peut utiliser un détecteur à capture d'électrons ECD, ou disposé en parallèle à un autre détecteur du type FID, ce qui nécessite alors un moyen de répartition des flux provenant du four, par exemple une vanne pilotée adaptée à supporter les hautes températures des flux.

Le conduit 12 amène une partie du flux à des moyens 13 d'analyse en continu des quantités de CO₂ et de CO produits par pyrolyse de l'échantillon. En sortie du four de pyrolyse, la division du flux est chauffée au moins à 360°C pour éviter les condensations de produits lourds.

Le conduit 12a amène au moins une partie du flux d'oxydation vers des moyens 13A d'analyse en continu des quantités de CO et CO₂ produits.

Des moyens de distribution 14 et 14a permettent d'utiliser seulement l'un ou l'autre des moyens d'analyse du CO et CO₂ pour le flux venant de la pyrolyse ou de l'oxydation. De préférence, pour des raisons de gain de temps opérationnel, les moyens 13a et 13 seront affectés à un seul moyen de chauffe. Les moyens d'analyse en continu sont par exemple des détecteurs à infrarouge.

Les cellules IR, étant spécifiques d'un gaz, peuvent mesurer en continu les concentrations de CO₂ et de CO dans les effluents au cours de la pyrolyse et de l'oxydation. Elles donnent accès à de nouveaux renseignements tels que la présence et la quantité de différents carbonates, les températures des maxima de dégagement, la forme des pics, la coupure entre le carbone minéral et le carbone organique et la répartition de chaque composé oxygéné dans les différentes réactions de craquage de la matière organique.

La longueur des cellules des détecteurs dépend de la sensibilité maximum demandée, donc de la concentration minimum qui doit être mesurée. Elle est fonction des quantités de CO ou CO₂ produites par l'échantillon (donc de sa masse), de la durée de l'analyse (donc des conditions de chauffage) et du débit du gaz vecteur qui est un facteur diluant.
* La cellule analysant le CO₂ mesure des concentrations maxima de 2% pour un débit variant de 50 à 200 ml. Cette plage est linéarisée sur quatre gammes à changement automatique:
   gamme 1 : 0 à 2% de CO₂
   gamme 2 : 0 à 1 % de CO₂
   gamme 3 : 0 à 0.5% de CO₂
   gamme 4 : 0 à 0.25% de CO₂
* La cellule analysant le CO mesure des concentrations maxima de 1% dans les mêmes conditions que la cellule CO₂. Les quatre gammes sont:
   gamme 1 : 0 à 1% de CO
   gamme 2 : 0 à 0.5% de CO
   gamme 3 : 0 à 0.25% de CO
   gamme 4 : 0 à 0.125% de CO

Les signaux récupérés sur les IR sont remis en forme pour obtenir des courbes à la même atténuation, digitalisés comme pour le signal FID.

Le dispositif comporte également des moyens de purification 15 et 15a des flux.

Les flèches 16 et 16a symbolisent les transferts des mesures vers les moyens électroniques de digitalisation.

De plus, le dispositif comporte un passeur 17 d'échantillons dont le bras 18 est adapté à déplacer la nacelle d'un échantillon entre trois positions possibles: le four de pyrolyse, le four d'oxydation, le magasin 19.

La mécanique du passeur est simplifiée de façon à ce que les déplacements puissent être effectués par des moteurs électriques pas à pas. Ainsi, toutes les possibilités de commande sont offertes et elles ne dépendent que du logiciel de fonctionnement. Il sera possible, par exemple, de charger les nacelles uniquement dans le four d'oxydation pour des études particulières. Une autre application possible sera de traiter thermiquement des échantillons dans un four et de les récupérer ensuite sur le support de nacelles ou magasin 19 pour les analyser selon le cycle désiré.

Le support des nacelles n'est pas linéaire mais circulaire: il prend la forme d'un carrousel qui occupe moins de place et qui permet d'accéder plus rapidement à la nacelle désirée par une marche avant ou arrière du chargeur. A chaque emplacement est assigné un numéro qui permet de programmer le passage des échantillons non seulement dans l'ordre chronologique du positionnement des nacelles sur le passeur mais aussi, soit en fonction des différents types de cycles ou d'analyses, soit en fonction des priorités d'analyse.

L'environnement du support de nacelles 19 est contrôlé thermiquement par des moyens 20 de régulation thermique, de façon à ce que les nacelles en attente soient maintenues à une température telles qu'il n'y ait pas de début de cycle de vaporisation, les nacelles étant sur le support. Les moyens 20 peuvent comporter des moyens d'isolation thermique vis-à-vis de la chaleur dégagée par le four et/ou des moyens de refroidissement local, par exemple une circulation d'un fluide de réfrigération, ou tout autre système.

Les nacelles peuvent comporter des opercules 21 ayant pour fonction de ne pas laisser s'évaporer une quantité de produit polluant léger, ce qui pourrait fausser les mesures d'évaluation. Dans ce cas, on prépare les nacelles sur le site. Avant l'introduction de la nacelle dans le four, il faut perforer l'opercule. Sur la figure 2, on a représenté schématiquement des moyens 22 liés au bras de chargement qui, par exemple, peuvent percuter l'opercule en même temps que le bras saisit une nacelle. Une autre solution, parmi d'autres à la portée d'un technicien, peut être de perforer l'opercule pendant la montée de la nacelle vers ou dans le four.

La figure 3 représente les séquences de température particulières conduisant à l'opération de pyrolyse d'un échantillon de sol pollué. Le nombre de ces séquences de température est au moins de quatre. Au temps t₀, l'échantillon est introduit dans le four déjà chauffé à la température initiale T₁. Cette valeur de température est inférieure à 120°C, préférentiellement proche de 80°C. La montée de l'échantillon dans le four est relativement rapide, ce qui porte très rapidement l'échantillon à la température intérieure du four. La durée t₁-t₀ de cette première étape de chauffe (conventionnellement nommée isotherme) est, par exemple, d'environ 10 minutes. A partir du temps t₁, on élève la température du four jusqu'à une température T₂ inférieure à 200°C et préférentiellement voisine de 180°C. La durée t₂-t₁ de montée en température est par exemple de l'ordre de 5 minutes de façon à obtenir un gradient de température compris entre 2 et 30°C/min, mais préférentiellement d'environ 20°C/min. La durée t₃-t₂ de la deuxième étape de chauffe est par exemple d'environ 15 minutes. A partir du temps t₃ débute une phase de pyrolyse programmée dans laquelle la montée en température est préférentiellement voisine de 20°C/min, jusqu'à la température T₃ correspondant au temps t₄. La valeur de cette température T₃ est d'environ 400°C, de préférence voisine de 370°C. La pyrolyse se poursuit ensuite jusqu'à atteindre la température T₄, au plus égale à 850°C. La montée programmée en température entre t₄ et t₅ n'est pas imposée, le seul facteur important est le temps passé en mesure et la capacité du four à monter en chauffe. La température T₄ peut donc être atteinte avec le même gradient de température que précédemment entre t₃ et t₄ ou selon un gradient de température différent.

Les résidus de pyrolyse sont ensuite brûlés dans le four d'oxydation entre 350°C et 850°C, à un gradient compris entre 25 et 50°C/min, de préférence entre 35 et 40°C.

Sur la figure 4, on peut voir une illustration de grandeurs représentatives de l'ensemble des hydrocarbures polluants pouvant être contenus dans un sol pollué selon la composition représentée sur la figure 1.

On constate que dans les conditions de chauffage décrits figure 3, l'échantillon de sol pollué peut donner en cours de programme de chauffe un premier pic Q₀, un pic Q₁, un pic Q₂ et un pic Q₃ respectivement référencés 30, 31, 32 et 33. Le pic Q₄, référencé 34, correspond à l'étape d'oxydation des résidus de pyrolyse.

Les pics Q₀, Q₁, Q₂ et Q₃ correspondent au dégagement des composés hydrocarbonés contenus dans l'échantillon de sol, compte tenu de la présence de produits polluants selon les classifications a, b, c, et d. On rappelle que:
- le type "a" correspond majoritairement aux composés hydrocarbonés légers, à point d'ébullition inférieur à 230°C, tels que les coupes de type essence, kérosène, et/ou les composés halogénés légers. Dans cette zone, le nombre d'atomes de carbone est majoritairement inférieur à 12;
- le type "b" correspond majoritairement aux composés hydrocarbonés, à point d'ébullition compris essentiellement entre 230°C et 400°C, tels que les coupes de type "gazole" et ou les composés halogénés de type polychlorobiphényl, etc.... Dans cette zone, le nombre d'atomes de carbone est majoritairement compris entre 13 et 25;
- le type "c" correspond majoritairement aux composés hydrocarbonés, à point d'ébullition compris entre 400 et 550°C, tels que les coupes pétrolières lourdes, les huiles lubrifiantes, etc.... Dans cette zone, le nombre d'atomes de carbone est majoritairement compris entre 25 et 40;
- le type "d" correspond majoritairement aux composés hydrocarbonés, à point d'ébullition sensiblement supérieur à 550°C, dont le nombre d'atomes de carbone est essentiellement supérieur à 40.

Bien entendu, en fonction de la nature du ou des polluants présents dans le sol, la caractérisation pourra ne comporter que trois, deux, voire un seul de ces quatre pics.

Des moyens de calcul, spécifiquement étalonnés, déterminent les quantités respectives des différents types de polluants en fonction de la forme, de l'amplitude desdits pics et du carbone organique restant après pyrolyse.

En effet, la pyrolyse des produits lourds s'accompagne généralement de la formation de coke. Le coke est ensuite brûlé dans le second four d'oxydation de l'appareil et le CO₂ et le CO produits sont mesurés et sommés donnant lieu à un pic représentatif d'une grandeur caractéristique Q₄ (34). On détermine ainsi le pourcentage de carbone organique résiduel dans l'échantillon, dénommé carbone organique résiduel Rc restant après pyrolyse, par opposition au carbone organique pyrolysé dénommé Pc.

Des analyses élémentaires effectuées sur le coke montrent que sa teneur en carbone organique est en moyenne de 90%.

Les différentes quantités Q₀, Q₁, Q₂, Q₃ et Q₄ sont exprimées en milligramme par gramme.

C'est à partir de ces quantités et de rapports spécifiques entre ces valeurs que l'on pourra juger de l'importance et de la nature de la contamination relevée dans le sol.

On a déterminé que certaines fonctions spécifiques des cinq grandeurs Q₀, Q₁, Q₂, Q₃ et Q₄ permettent de définir les types et les quantités de polluants. En particulier, on a déterminé qu'à partir de deux fonctions du type f(Q₀, Q₁, Q₂) et g(Q₀, Q₁, Q₂, Q₃ et Q₄), il était possible de caractériser la plupart des polluants.

Plus précisément: la fonction f peut avoir de préférence la forme suivante: Q₀/(Q₀+Q₁+Q₂), et g de préférence la forme: (Q₃+Q₄)/(Q₀+Q₁+Q₂).
Pour un polluant de type "a", le rapport Q₀/(Q₀+Q₁+Q₂) est compris entre 0,5 et 1 et le rapport (Q₃+Q₄)/(Q₀+Q₁+Q₂) est voisin de zéro,
Pour un polluant de type "b", le rapport Q₀/(Q₀+Q₁+Q₂) est compris entre 0,05 et 0,5 et le rapport (Q₃+Q₄)/(Q₀+Q₁+Q₂) est voisin de zéro,
Pour un polluant de type "c", le rapport Q₀/(Q₀+Q₁+Q₂) est voisin de zéro, et le rapport (Q₃+Q₄)/(Q₀+Q₁+Q₂) est compris entre 0 et 5,
Pour un polluant de type "d", le rapport Q₀/(Q₀+Q₁+Q₂) est voisin de zéro, et le rapport (Q₃+Q₄)/(Q₀+Q₁+Q₂) est supérieur à 10.

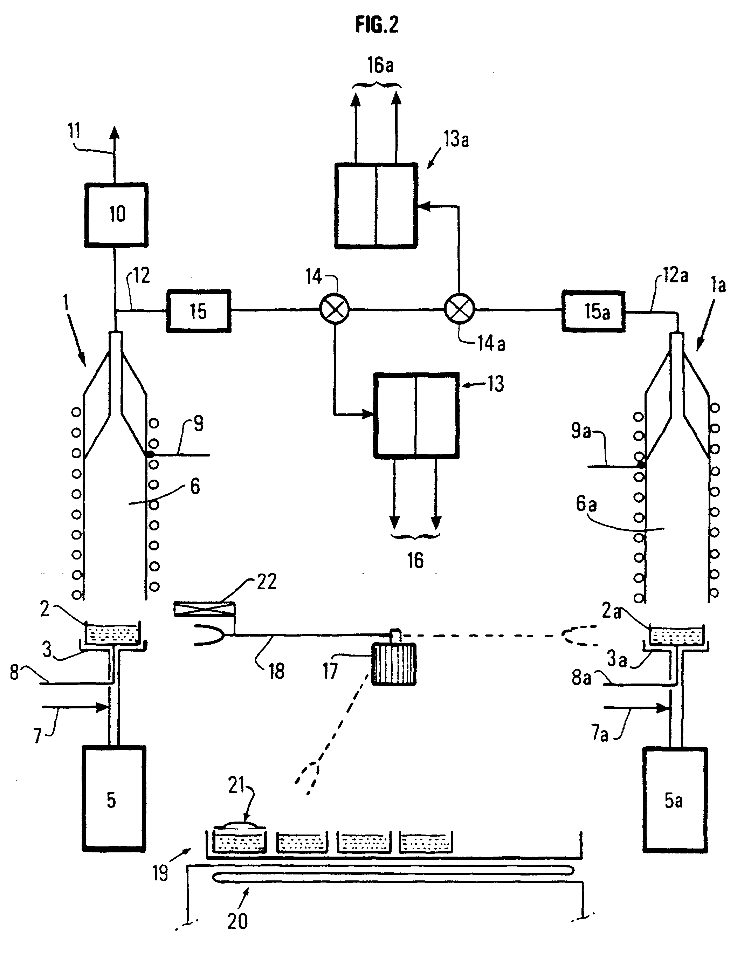

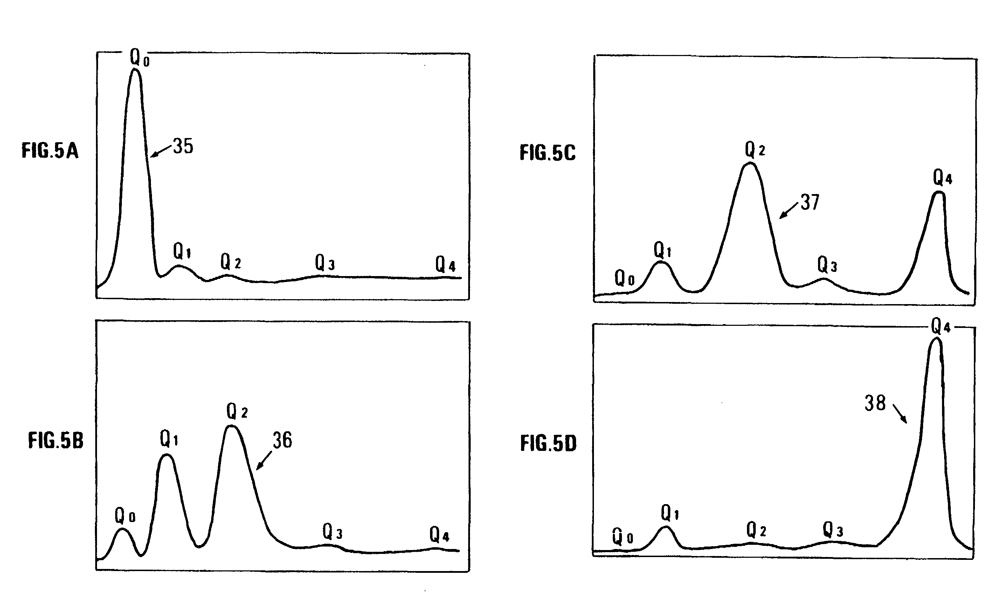

Les figures 5A, 5B, 5C et 5D montrent quatre exemples d'application de la méthode sur des échantillons de sols pollués par différents types d'hydrocarbures. Les graphiques 35, 36, 37, et 38 représentent les grandeurs associées à différents types d'hydrocarbures polluants. Le programme de pyrolyse, selon la figure 3 est le suivant:
- T1=100°C pendant 10 minutes;
- T2=180°C (gradient de 20°C/min);
- Isotherme de 2 minutes à 180°C;
- T3=370°C (gradient de 20°C/min);
- T4=850°C (gradient de 20°C/min);
- Oxydation entre 350 et 800°C (gradient 35°C/min).

La figure 5A illustre une pollution due à un "jet fuel", c'est-à-dire un carburant aviation appartenant au type a. Dans cet exemple, le rapport Q₀/(Q₀+Q₁+Q₂) est voisin de 0,86 et le rapport (Q₃+Q₄)/(Q₀+Q₁+Q₂) est voisin de zéro.

La figure 5B illustre une pollution due à un gazole routier, appartenant au type b. Dans cet exemple, le rapport Q₀/(Q₀+Q₁+Q₂) est voisin de 0,17 et le rapport (Q₃+Q₄)/(Q₀+Q₁+Q₂) est voisin de zéro.

La figure 5C illustre une pollution due à une huile lubrifiante, appartenant au type c. Dans cet exemple, le rapport Q₀/(Q₀+Q₁+Q₂) est voisin de 0 et le rapport (Q₃+Q₄)/(Q₀+Q₁+Q₂) est voisin de 0,4.

La figure 5D illustre une pollution due à un résidu de distillation de houille, appartenant au type d. Dans cet exemple, le rapport Q₀/(Q₀+Q₁+Q₂) est voisin de 0 et le rapport (Q₃+Q₄)/(Q₀+Q₁+Q₂) est voisin de 13.

## Revendications

1. Méthode d'évaluation d'au moins une caractéristique de pollution de sols naturels par des composés hydrocarbonés, à partir d'un échantillon desdits sols, méthode dans laquelle l'échantillon est chauffé dans un premier temps en atmosphère non oxydante puis, dans un second temps, en atmosphère oxydante, ladite méthode comporte les étapes suivantes :
a) on élève rapidement la température de l'échantillon à une première valeur de température comprise entre 80 et 120°C et pendant une durée déterminée,
b) à partir de la première valeur de température, on élève la température de l'échantillon jusqu'à une seconde valeur de température inférieure à 200°C selon un gradient de température compris entre 2 et 30°C/min et on demeure à cette température pendant une durée déterminée,
c) on élève la température de la seconde valeur à une troisième valeur de température inférieure à 500°C, selon un gradient de température compris entre 10 et 40°C/min,
d) on élève la température de l'échantillon de la troisième valeur à une quatrième valeur au plus égale à 850°C,
e) on détermine à partir des quatre étapes précédentes, quatre grandeurs Q₀, Q₁, Q₂, Q₃ représentatives de la nature et de la quantité des composés hydrocarbonés contenus dans ledit échantillon,
f) après l'élévation de température à la quatrième valeur, on brûle, en atmosphère oxydante, les résidus dudit échantillon depuis une température supérieure à 350°C jusqu'à une température au plus égale à 850°C, selon un gradient de température compris entre 20 et 50°C/min,
g) on détermine une quantité Q₄ représentative de la quantité de carbone organique résiduel après la pyrolyse,
h) on déduit des cinq grandeurs Q₀, Q₁, Q₂, Q₃ et Q₄ au moins une caractéristique de pollution dudit échantillon.

2. Méthode selon la revendication 1, dans laquelle on évalue la quantité Q de produit polluant contenu dans le sol, telle que:
Q = Q₀ + Q₁ + Q₂ + Q₃ + kQ₄
avec k prenant l'une des valeurs de 10 à 11,5.

3. Méthode selon l'une des revendications 1 ou 2, dans laquelle on détermine au moins une fonction liant au moins une grandeur parmi le groupe constitué par Q₀, Q₁, Q₂, Q₃, et Q₄, et dans laquelle on distingue le type "a", "b", "c", ou "d" du polluant en fonction d'une valeur spécifique de ladite fonction pour un échantillon donné.

4. Méthode selon la revendication 3, dans laquelle on distingue le type "a", "b", "c", ou "d" du polluant à l'aide d'au moins l'un des rapports spécifiques suivants :
- pour un polluant de type "a", le rapport Q₀/(Q₀+Q₁+Q₂) est compris entre 0,5 et 1 et le rapport (Q₃+Q₄)/(Q₀+Q₁+Q₂) est voisin de zéro,
- pour un polluant de type "b", le rapport Q₀/(Q₀+Q₁+Q₂) est compris entre 0,05 et 0,5 et le rapport (Q₃+Q₄)/(Q₀+Q₁+Q₂) est voisin de zéro,
- pour un polluant de type "c", le rapport Q₀/(Q₀+Q₁+Q₂) est voisin de zéro, et le rapport (Q₃+Q₄)/(Q₀+Q₁+Q₂) est compris entre 0 et 5,
- pour un polluant de type "d", le rapport Q₀/(Q₀+Q₁+Q₂) est voisin de zéro, et le rapport (Q₃+Q₄)/(Q₀+Q₁+Q₂) est supérieur à 10.

5. Dispositif pour l'évaluation d'au moins une caractéristique de pollution de sols naturels par des composés hydrocarbonés, à partir d'un échantillon desdits sols placé dans une nacelle, ledit dispositif comportant un premier moyen de chauffe dudit échantillon sous atmosphère non oxydante, des moyens de mesure de la quantité des composés hydrocarbonés dégagés à la suite de l'introduction de l'échantillon dans ledit premier moyen de chauffe, des moyens de transfert de l'échantillon dans un second moyen de chauffe sous atmosphère oxydante, des moyens de mesure de la quantité de CO₂ contenue dans les effluents évacués des deux moyens de chauffe, lesdits moyens de mesure du CO₂ comportant une cellule de mesure en continu du CO₂ à chaque instant du cycle de chauffe du premier et du second moyen de chauffe et comportant des moyens de mesure de la quantité de CO contenue dans les effluents évacués par les deux moyens de chauffe, **caractérisé en ce qu'**il comporte des moyens de détermination du type de polluant, en accord avec la méthode de la revendication nº 1.

6. Dispositif selon la revendication 5, dans lequel il comporte des moyens de contrôle de la température des nacelles en attente d'introduction dans lesdits moyens de chauffe.

7. Dispositif selon la revendication 6, dans lequel lesdits moyens de contrôle de la température comporte l'un au moins des éléments suivants: une ventilation, une circulation d'un fluide réfrigérant, une isolation.

8. Dispositif selon l'une des revendications 5 à 7, dans lequel les nacelles comportent des moyens d'obturation.

9. Dispositif selon la revendication 8, dans lequel il comporte des moyens d'ouverture des nacelles avant introduction dans les moyens de chauffe.

## Claims

1. A method of evaluating at least one pollution characteristic of natural soils contaminated by hydrocarbon compounds, from a sample of the said soils, a method in which the sample is first heated in a non-oxidising atmosphere, then in an oxidising atmosphere, the said method including the following stages:
a) the temperature of the sample is rapidly raised to a first temperature value of between 80 and 120 C for a certain period.
b) starting from the first temperature value, the temperature of the sample is raised to a second level below 200 C at a temperature gradient ranging between 2 and 30 C per minute and this temperature is maintained for a certain period.
c) the temperature is raised from the second value to a third value below 500 C at a temperature gradient ranging between 10 and 40 C per minute.
d) the temperature of the sample is raised from the third value to a fourth value not exceeding 850 C.
e) four values Q₀, Q₁, Q₂ and Q₃, representative of the nature and quantity of hydrocarbon compounds contained in the said sample, are determined from the previous four stages.
f) after raising the temperature to the fourth value, the residues of the said sample are burned in an oxidising atmosphere from a temperature greater than 350 C to a temperature not exceeding 850 C at a temperature gradient ranging between 20 and 50 C per minute.
g) a value Q₄ representative of the quantity of residual organic carbon after pyrolysis is determined.
h) at least one pollution characteristic of the said sample is deduced from the five values Q₀, Q₁, Q₂, Q₃ and Q₄

2. A method according to Claim 1 in which the quantity Q of pollutant contained in the soil is evaluated such that:
Q = Q₀ + Q₁ + Q₂ + Q₃ + kQ₄
with k taking a value ranging from 10 to 11.5.

3. A method according to either of Claims 1 and 2 in which at least one function relating at least one quantity from the group comprising Q₀, Q₁, Q₂, Q₃ and Q₄ is determined and in which the type "a", "b", "c" or "d" of the pollutant is distinguished according to a specific value of the said function for a given sample.

4. A method according to Claim 3, wherein the type "a", "b", "c" or "d" of the pollutant is distinguished by means of at least one of the specific ratios as follows:
- for a pollutant of type "a", the ratio Q₀/(Q₀+Q₁+Q₂) ranges between 0.5 and 1 and the ratio (Q₃+Q₄)/(Qo+Q₁+Q₂) is close to zero
- for a pollutant of type "b", the ratio Q₀/(Q₀+Q₁+Q₂) ranges between 0.05 and 0.5, and the ratio (Q₃+Q₄)/(Q₀+Q₁+Q₂) is close to zero
- for a pollutant of type "c", the ratio Q₀/(Q₀+Q₁+Q₂) is close to zero and the ratio (Q₃+Q₄)/(Q₀+Q₁+Q₂) ranges between 0 and 5
- for a pollutant of type "d", the ratio Q₀/(O₀+Q₁+Q₂) is close to zero and the ratio (Q₃+Q₄)/(Q₀+Q₁+Q₂) is greater than 10.

5. A system for evaluating at least one pollution characteristic of natural soils contaminated by hydrocarbon compounds, from a sample of the said soils placed in a boat, the said system comprising a first device for heating the said sample in a non-oxidising atmosphere, a device for measuring the quantity of hydrocarbon compounds released after feeding the sample into the said first heating device, a means of transferring the sample into a second heating device in an oxidizing atmosphere, a means of measuring the amount of CO₂ contained in the effluents discharged from the two heating devices, the said means of measuring CO₂ comprising a cell for measuring continuously the CO₂ throughout the heating cycle of the first and second heating devices and including a means of measuring the quantity of CO contained in the effluents discharged from the two heating devices, **characterised by** the fact that it comprises a means of determining the pollutant type in accordance with the method of Claim 1.

6. A system according to Claim 5, comprising a means of controlling the temperature of the boats waiting to be introduced into the said heating devices.

7. A system according to Claim 6, wherein the said means of temperature control comprises at least one of the following elements: ventilation, circulation of a cooling fluid, insulation.

8. A system according to any one of Claims 5 to 7, wherein the boats comprise a means of closure.

9. A system according to Claim 8, comprising a means of opening the boats prior to introducing them into the heating devices.

## Patentansprüche

1. Verfahren zur Bewertung wenigstens einer Verunreinigungscharakteristik natürlicher Böden durch kohlenwasserstoffhaltige Zusammensetzungen, ausgehend von einer Probe dieser Böden, Verfahren, bei dem die Probe in einem ersten Zeitraum in nicht-oxidierender Atmosphäre, in einem zweiten Zeitraum in oxidierender Atmosphäre erwärmt wird, wobei dieses Verfahren die folgenden Stufen umfasst:
a) man erhöht schnell die Temperatur der Probe auf einen ersten Temperaturwert zwischen 80 und 120°C und während einer bestimmten Dauer,
b) ausgehend von dem ersten Temperaturwert erhöht man die Temperatur der Probe bis auf einen zweiten Temperaturwert unterhalb 200°C gemäß einem Temperaturgradienten zwischen 2 und 30°C/min und man bleibt bei dieser Temperatur eine vorbestimmte Zeitdauer lang,
c) man erhöht die Temperatur des zweiten Werts auf einen dritten Temperaturwert unterhalb 500°C gemäß einem Temperaturgradienten zwischen 10 und 40°C/min,
d) man erhöht die Temperatur der Probe des dritten Wertes auf einen vierten Wert der höchstens gleich 850°C ist,
e) man bestimmt, ausgehend von den vier vorhergehenden Stufen vier Größen Q₀, Q₁, Q₂, Q₃, die repräsentativ für die Natur und die Menge der in dieser Probe enthaltenen kohlenwasserstoffhaltigen Verbindungen ist,
f) nach Erhöhung der Temperatur auf diesen vierten Wert verbrennt man in oxidierender Atmosphäre die Rückstände dieser Probe ab einer Temperatur oberhalb 350°C bis auf eine Temperatur höchstens gleich 850°C gemäß einem Temperaturgradienten zwischen 20 und 50°C/min,
g) man bestimmt eine Menge Q₄, die repräsentativ für die Menge an verbleibendem organischen Kohlenstoff nach der Pyrolyse ist und
h) man leitet aus den vier Werten Q₀, Q₁, Q₂, Q₃ und Q₄ wenigstens eine Charakteristik für die Verunreinigung dieser Probe her.

2. Verfahren nach Anspruch 1, bei dem man die Menge Q an im Boden enthaltenem verunreinigenden Produkt bewertet, derart, dass Q = Q₀+Q₁+Q₂+Q₃+ kQ₄, wobei k einen der Werte 10 bis 11,5 annimmt.

3. Verfahren nach einem der Ansprüche 1 oder 2, bei dem man wenigstens eine Funktion bestimmt, die wenigstens eine Größe bindet, die aus der Gruppe gewählt ist, die gebildet ist Q₀, Q₁, Q₂, Q₃ und Q₄ und wobei man den Typ "a", "b", "c" oder "d" der Verunreinigung als Funktion eines spezifischen Wertes dieser Funktion für eine gegebene Probe unterscheidet.

4. Verfahren nach Anspruch 3, bei dem man den Typ "a", "b", "c" oder "d" der Verunreinigung mit Hilfe wenigstens eines der spezifischen Verhältnisse unterscheidet:
- für eine Verunreinigung vom Typ "a" liegt das Verhältnis Q₀/ (Q₀+Q₁+Q₂) zwischen 0,5 und 1 und das Verhältnis (Q₃+Q₄)/(Q₀+Q₁+Q₂) nahe null,
- für eine Verunreinigung vom Typ "b" liegt das Verhältnis von Q₀/(Q₀+Q₁+Q₂) zwischen 0,05 und 0,5 und das Verhältnis (Q₃+Q₄)/(Q₀+Q₁+Q₂) nahe null,
- für eine Verunreinigung vom Typ "c" liegt das Verhältnis von Q₀/(Q₀+Q₁+Q₂) nahe null und das Verhältnis (Q₃+Q₄)/(Q₀+Q₁+ Q₂) zwischen 0 und 5,
- für eine Verunreinigung vom Typ "d" liegt das Verhältnis von Q₀/(Q₀+Q₁+Q₂) nahe null und das Verhältnis (Q₃+Q₄)/(Q₀+Q₁+ Q₂) über 10.

5. Vorrichtung zur Bewertung wenigstens einer Charakteristik der Verunreinigung natürlicher Böden durch kohlenwasserstoffhaltige Verbindungen, ausgehend von einer Probe dieser Böden, die in ein Schiffchen eingesetzt ist, wobei die Vorrichtung ein erstes Heizmittel für diese Probe unter nicht-oxidierender Atmosphäre, Messmittel für die Menge der kohlenwasserstoffhaltigen anschließend an die Einführung der Probe in dieses erste Heizmittel freigesetzten Verbindungen, Mittel zur Überführung der Probe in ein zweites Heizmittel unter oxidierender Atmosphäre, Mittel zum Messen der Menge von in den Abströmen enthaltenem CO₂, wobei diese aus den beiden Heizmitteln abgezogen wurden, und die Mittel zum Messen des CO₂ eine Zelle zur kontinuierlichen Messung des CO₂ in jedem Augenblick des Heizzyklus des ersten und des zweiten Heizmittels aufweisen, und Mittel zum Messen der Menge von CO umfassen, das in den aus den beiden Heizmitteln abgezogenen Abströmen enthalten ist, **dadurch gekennzeichnet, dass** sie Mittel zur Bestimmung des Verunreinigungstyps in Übereinstimmung mit dem Verfahren des Anspruchs 1 umfasst.

6. Vorrichtung nach Anspruch 5, wobei sie Mittel zur Regelung der Temperatur der in Wartestellung zur Einführung in diese Heizmittel begriffenen Schiffchen regelt.

7. Vorrichtung nach Anspruch 6, bei dem diese Regelmittel für die Temperatur wenigstens eines der folgenden Elemente umfassen: eine Ventilation, eine Zirkulation für ein Kühlfluid sowie eine Isolation.

8. Vorrichtung nach einem der Ansprüche 5 bis 7, wobei die Schiffchen Schließmittel umfassen.

9. Vorrichtung nach Anspruch 8, wobei sie Mittel zum Öffnen der Schiffchen vor dem Einführen in die Heizmittel umfasst.
